(19) Europäisches Patentamt
European Patent Office
Office européen des brevets

(11) Veröffentlichungsnummer : **0 367 050 B1**

# (12) EUROPÄISCHE PATENTSCHRIFT

(45) Veröffentlichungstag der Patentschrift : **09.09.92 Patentblatt 92/37**

(51) Int. Cl.[5] : **C07C 51/60,** C07C 53/42, C07C 57/64

(21) Anmeldenummer : **89119548.9**

(22) Anmeldetag : **21.10.89**

## (54) Verfahren zur Herstellung von Carbonsäurechloriden.

(30) Priorität : **31.10.88 DE 3836967**

(43) Veröffentlichungstag der Anmeldung : **09.05.90 Patentblatt 90/19**

(45) Bekanntmachung des Hinweises auf die Patenterteilung : **09.09.92 Patentblatt 92/37**

(84) Benannte Vertragsstaaten : **BE CH DE ES FR GB IT LI NL**

(56) Entgegenhaltungen :
**EP-A- 0 031 504**
**DE-A- 2 057 956**

(73) Patentinhaber : **BASF Aktiengesellschaft**
**Carl-Bosch-Strasse 38**
**W-6700 Ludwigshafen (DE)**

(72) Erfinder : **Hohmann, Andreas, Dr.**
**Sinsheimer Strasse 22**
**W-6700 Ludwigshafen (DE)**
Erfinder : **Freudenberg, Enrique, Dr.**
**Bayernstrasse 4**
**W-6707 Schifferstadt (DE)**
Erfinder : **Reuther, Wolfgang, Dr.**
**Am Pferchelhang 16**
**W-6900 Heidelberg (DE)**
Erfinder : **Mayer, Kurt, Dr.**
**Friedensstrasse 95**
**W-6700 Ludwigshafen (DE)**

EP 0 367 050 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Carbonsäurechloride lassen sich gut durch Umsetzung der entsprechenden Carbonsäuren mit Phosgen herstellen. Die Reaktion bedarf der Katalyse. Als Katalysatoren kommen z.B. Carboxamide, vorzugsweise N-Alkylformamide zum Einsatz (DE-A-34 39 937).

Die Spanne der Alkylgröße reicht bei den N,N-Dialkylformamiden von Dimethylformamid bis zu Formamiden mit 30 C-Atomen (EP-A-0 050 779, DE-A-29 50 155, DE-A-19 31 074).

Durch die Wahl des Katalysatorsystems wird der Ablauf der Phosgenierung einer Carbonsäure zum Carbonsäurechlorid sowie die Aufarbeitung des Ansatzes entscheidend beeinflußt.

Alternativ zur Filtration von teerhaltigen Rohprodukten wäre in einigen Fällen auch eine destillative Aufarbeitung des katalysatorhaltigen Produktes denkbar. Die Destillation der entstandenen Säurechloride ist aber nicht nur ein energie- und zeitaufwendiger Vorgang, sondern birgt auch noch eine Reihe weiterer Nachteile.

Viele längerkettige Säurechloride lassen sich nicht ohne teilweise Zersetzung destillieren. Weiter ist bekannt, daß durch Zersetzung des im Destillationssumpf befindlichen Katalysators die destillierten Produkte verunreinigt werden können. Größere Mengen an Katalysatorrückstand stellen bei der Destillation ein Sicherheitsrisiko dar, weil in der Hitze die Gefahr einer spontanen Zersetzung besteht.

Bei der Produktaufarbeitung verunreinigter Ansätze vermindert sich sowohl durch Filtration als auch durch Destillation die Aktivität des Katalysators stark. In den meisten Fällen wird der eingesetzte Katalysator unbrauchbar, er kann also nicht wiederverwendet werden.

Sowohl die Destillation als auch die Filtration von katalysatorhaltigen Carbonsäurechloriden stellen also unvorteilhafte Aufarbeitungsmethoden dar. Wegen des durch die Aufarbeitung bedingten Katalysatorverlustes muß dessen Aufwandmenge so gering wie möglich ausfallen.

In der DE-A-29 50 155 wird als Katalysator Diisobutylformamid verwendet, das in jeder Phase der Umsetzung im Reaktionsansatz löslich ist. Soll auf eine abschließende Destillation des Säurechlorids verzichtet werden, muß aus Gründen der Produktreinheit der Anteil des löslichen Katalysators auf ein Minimum beschränkt werden. Auch bei diesem Katalysatorsystem ist eine Wiederverwendung des Katalysators ausgeschlossen, da er mit dem Produkt ausgetragen wird.

Es ist auch bekannt, daß die Umsetzungen mit Phosgen um so effektiver ablaufen, je größer der Katalysatoranteil ist. Umgekehrt bedingen geringe Katalysatormengen entweder eine schlechte Ausnutzung des eingegasten Phosgens oder lange Eingaszeiten.

In der DE-A-22 40 883 wird die Herstellung von Carbonsäurechloriden mit äquimolaren Mengen Carbonsäure und Katalysator beschrieben. Zur Abtrennung und Wiedergewinnung der großen Katalysatormenge ist allerdings die abschließende Zugabe des 3- bis 4-fachen Reaktionsvolumens an Benzol mit anschließender Destillation der benzolischen Produktlösung notwendig.

Den Einsatz großer Mengen Katalysator beschreibt auch JP-10 613/68 bei der Herstellung von Linolsäurechlorid mit 10 bis 50 Mol.% Dimethylformamid, aber auch 1 bis 10 Äquivalenten Dimethylformamid, bezogen auf eingesetzte Linolsäure. Das entstandene Säurechlorid muß destilliert und zum Teil durch eine Behandlung mit Aktivkohle zusätzlich gereinigt werden. Die erneute Verwendung der großen Katalysatormengen ist nicht vorgesehen.

Bei der Synthese von Carbonsäuren mit Phosgen zu den entsprechenden Säurechloriden stellt sich bekanntermaßen das Problem, überschüssiges Phosgen aus dem rohen Säurechlorid zu entfernen.

Nach dem Stand der Technik kann phosgenhaltiges Carbonsäurechlorid durch mehrstündiges Strippen mit Stickstoff und/oder durch Anlegen eines leichten Vakuums von Phosgen befreit werden. Dieser Vorgang ist zeitraubend und verschlechtert die Raum-Zeit-Ausbeute des Verfahrens erheblich.

In der DE-A-29 50 155 wird das überschüssige Phosgen mit dem ersten Teil des destillierten Säurechlorids überdestilliert. Neben einer auch hier zu beobachtenden Verschlechterung der Raum-Zeit-Ausbeute erfordert diese Vorgehensweise zusätzlichen apparativen und analytischen Aufwand.

Aus der DE-A-22 40 883 ist eine Aufarbeitung bekannt, bei der vor der Destillation die verdünnte Reaktionslösung kurz mit Eiswasser gewaschen wird. Angesichts der Hydrolyseempfindlichkeit von Carbonsäurechloriden ist dieses Verfahren für den technischen Maßstab problematisch.

Auch bei dem aus JP 10613/68 bekannten Verfahren muß überschüssiges Phosgen durch eine destillative Aufarbeitung des rohen Säurechlorids entfernt werden.

Der Erfindung lag nun die Aufgabe zugrunde, ein Verfahren zur Herstellung von Carbonsäurechloriden zu finden, das den vorgenannten Nachteilen abhilft.

Demgemäß wurde ein Verfahren zur Herstellung von Carbonsäurechloriden der allgemeinen Formel I

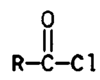

(I),

in der R für $C_8$- bis $C_{30}$-Alkyl, $C_8$- bis $C_{30}$-Alkenyl und $C_8$- bis $C_{30}$-Alkinyl steht, aus Carbonsäuren der allgemeinen Formel II

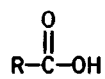

(II),

in der R die oben genannten Bedeutungen hat, und Phosgen, $COCl_2$ (III), in Gegenwart eines Katalysator-Addukts aus Phosgen und N,N-Dialkylformamid der allgemeinen Formel IV

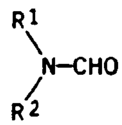

(IV),

in der $R^1$ und $R^2$ unabhängig voneinander $C_1$- bis $C_3$-Alkyl bedeuten, wobei das Reaktionsprodukt I durch Phasentrennung erhalten wird, gefunden, welches dadurch gekennzeichnet ist, daß man II und III in im wesentlichen äquimolaren Mengen verwendet und die das Katalysator-Addukt enthaltene Phase wiederverwendet.

Die Carbonsäurechloride sind nach folgender Methode erhältlich:

Zum vorgelegten Reaktionsgemisch, bestehend aus einer Carbonsäure II und dem Addukt aus Phosgen und N,N-Dialkylformamid der Formel IV, wird flüssiges oder gasförmiges Phosgen gegeben. Der Zeitbedarf für das Eingasen des Phosgens kann dabei auf 3 bis 4 Stunden begrenzt werden, wobei das Phosgen praktisch quantitativ ausgenutzt wird. Anschließend läßt man 1 bis 2 Stunden absitzen und trennt die Phasen.

Bei dieser Umsetzung verwendet man 5 bis 200 Mol-%, vorzugsweise 10 bis 100 Mol-%, besonders bevorzugt 10 bis 30 Mol-% des Addukts aus Phosgen und N,N-Dialkylformamid IV, bevorzugt eines 1:1-Adduktes, gegebenenfalls in überschüssigem N,N-Dialkylformamid,, bezogen auf die eingesetzte Carbonsäure II.

Die Phosgenmenge III ist im wesentlichen äquimolar zur Carbonsäure II.

Das Phosgen kann mit Inertgasen vermischt sein. Die Reaktion kann sowohl bei Normaldruck als auch bei erniedrigtem oder erhöhtem Druck durchgeführt werden. Die Reaktionstemperatur kann zwischen 0 und 200°C liegen. Bevorzugt ist der Bereich von 30 bis 100°C, besonders bevorzugt eine Temperatur von 60 bis 80°C.

Das Reaktionsgemisch kann auch bei Bedarf mit Lösungsmitteln versetzt sein. Diese müssen sich unter den Reaktionsbedingungen inert verhalten, wie z.B. gesättigte aliphatische Kohlenwasserstoffe, Ether, Acetonitril, Toluol, Benzol oder Cyclohexan.

Bei einer Temperatur, die zwischen 0 und 150°C, bevorzugt zwischen 20 und 50°C liegt, wird die untere, vom Katalysator gebildete Phase von der oberen Produktphase abgetrennt. Dazu sind mehrere Varianten möglich. So kann z.B. der Katalysator bis zur erneuten Verwendung in ein Vorratsgefäß abgelassen werden. Es ist aber auch möglich, das Carbonsäurechlorid, d.h. die obere Phase, über ein Steigrohr vom Katalysator abzuheben und diesen bis zur nächsten Umsetzung im Reaktor zu belassen.

Das Carbonsäurechlorid fällt quantitativ und in hoher Reinheit an. Es kann ohne weitere Reinigung, wie z.B. Destillation oder Filtration verwendet werden. Ein besonderer Vorteil des erfindungsgemäßen Verfahrens besteht darin, daß das Carbonsäurechlorid am Ende der Umsetzung phosgenfrei vorliegt. Dadurch können alle Maßnahmen zum Entphosgenieren des rohen Säurechlorids entfallen.

Besonders vorteilhaft beim erfindungsgemäßen Verfahren ist auch die völlig unproblematische Rückgewinnung und Wiederverwendung des Katalysators.

Das erfindungsgemäße Verfahren zur Herstellung von Säurechloriden aus aliphatischen Carbonsäuren eignet sich vor allem für Monocarbonsäuren, d.h. zur Herstellung von Verbindungen mit der allgemeinen Formel RCOX, wobei R eine aliphatische Kohlenwasserstoffgruppe darstellt und X für Chlor steht. Die aliphatische Gruppe kann geradkettig oder verzweigt, gesättigt, olefinisch oder acetylenisch ungesättigt sein. Besonders bevorzugt sind aliphatische Carbonsäuren mit 8 bis 30, insbesondere 12 bis 22 Kohlenstoffatomen.

Als N,N-Dialkylformamide eignen sich Dimethyl-, Ethyl-methyl-, Methyl-n-propyl-, Methyl-iso-propyl-, Diethyl-, Ethyl-n-propyl, Ethyl-isopropyl-, Di-n-propyl-, n-Propyl-iso-propyl- und Di-iso-propyl-formamid, bevorzugt sind Dimethyl- und Diethylformamid, besonders bevorzugt ist Diethylformamid.

Beispiele

Beispiel 1

In einem thermostatisierten 5-l-Reaktor werden 2746 g (10 mol) technische Stearinsäure bei 70°C vorgelegt und 202 g (2 mol) Diethylformamid (DEF) zugefügt. Anschließend werden gleichmäßig über einen Zeitraum von 2,5 Stunden unter gutem Rühren 1188 g (12 mol) Phosgen eingegast. Die Innentemperatur beträgt während der Phosgenzufuhr 70 bis 75°C. Das Abgas der Reaktion wird direkt über einen Wascher geleitet, in dem unverbrauchtes Phosgen hydrolysiert wird.

Nach dem Ende der Phosgenzugabe wird 0,5 Stunden nachgerührt, der Rührer abgeschaltet und der Reaktionsansatz in einen Scheidetrichter überführt. Der Dampfraum über dem Ansatz ist phosgenfrei (Prüfröhrchen der Fa. Dräger).

Nach 2 Stunden bei 25°C wird aus dem zweiphasigen Reaktionsansatz die untere Katalysatorphase (342 g aktiviertes DEF) abgetrennt. Die obere Phase enthält 2900 g Stearinsäurechlorid (9,89 mol = 98,9 % Ausbeute, bezogen auf eingesetzte technische Stearinsäure) mit einer Reinheit von 95 % (IR-spektroskopisch bestimmt). Die Iodfarbzahl (IFZ) des Säurechlorids beträgt 10. Sowohl das Säurechlorid als auch der Katalysator sind phosgenfrei.

Beispiel 2 bis 10

Um die Leistungsfähigkeit des erfindungsgemäßen Verfahrens zu ermitteln, wird Beispiel 1 mehrfach reproduziert. Als Katalysator kommt jeweils die Katalysatorphase des vorhergegangenen Versuches zum Einsatz.

Beispiel 2

Nach der im Beispiel 1 beschriebenen Versuchsvorschrift werden 2746 g (10 mol) technische Stearinsäure und die im Beispiel 1 erhaltene Katalysatorphase (342 g) vorgelegt. Anschließend werden 1089 g (11 mol) Phosgen eingegast. Die Produktphase enthält 2930 g (99,9 % Ausbeute) Stearinsäurechlorid, Reinheit 97 % IR); IFZ: 30.

Die Durchführung der Beispiele 3 bis 10 gestaltet sich analog zu Beispiel 2

| Bsp. | Säure (mol) | Phosgen (mol) | Ausbeute | Reinheit (IR) | IFZ |
|---|---|---|---|---|---|
| 3 | 10,0 | 10,0 | 99 % | 97 % | 20 |
| 4 | 10,0 | 10,0 | 100 % | 95 % | 10 |
| 5 | 10,0 | 10,0 | 100 % | 96 % | 10 |
| 6 | 10,0 | 10,5 | 100 % | 96 % | 10 |
| 7 | 10,0 | 10,0 | 100 % | 94 % | 25 |
| 8 | 10,0 | 10,0 | 100 % | 96 % | 15 |
| 9 | 10,0 | 10,0 | 100 % | 96 % | 15 |
| 10 | 10,0 | 10,5 | 100 % | 97 % | 15 |

Alle Säurechloride und Katalysatoren fallen phosgenfrei an.

Beispiel 11

In einem Reaktor werden bei 65°C 542 g (2,0 mol) technische Stearinsäure und 44 g Di-isobutylformamid (0,28 mol) vorgelegt und in die Schmelze unter gutem Rühren innerhalb von 2,5 Stunden 208 g (2,1 mol) Phosgen eingegast. Die Innentemperatur wird bei 65°C gehalten. Anschließend wird 0,5 Stunden bei 60°C nachgerührt.

Die Umsetzung der Stearinsäure ist vollständig. Der Reaktionsansatz ist phosgenfrei. Auch nachdem der Ansatz auf 20°C abgekühlt ist, bleibt der Katalysator im rohen Säurechlorid gelöst. Der Austrag an Stearinsäure und darin enthaltendem Katalysator beträgt 624 g (maximal mögliche Ausbeute an Stearinsäurechlorid: 579 g). Der Einheitsgrad des Säurechlorids beträgt 84 % (IR). Das Produkt ist von brauner Farbe (IFZ: 110).

Beispiel 12

In einem thermostatisierten 5-l-Reaktor werden 2028 g (6 mol) technische Behensäure bei 73°C vorgelegt und 223 g (ca. 1,2 mol) aktiviertes Diethylformamid zugefügt. Anschließend werden gleichmäßig über einen Zeitraum von 2,75 Stunden unter gutem Rühren 596 g (6,0 mol) Phosgen eingegast. Die Innentemperatur beträgt während der Phosgenzufuhr 76 bis 80°C. Das Abgas der Reaktion wird direkt über einen Wascher abgeleitet. Nach dem Ende der Umsetzung ist der Dampfraum über dem Reaktionsansatz phosgenfrei.

Danach wird 1,0 Stunden bei 77°C nachgerührt, der Rührer abgeschaltet und der Reaktionsansatz auf 42°C abgekühlt. Bei dieser Temperatur wird nach 1,5 Stunden aus dem zweiphasigen Reaktionsansatz die untere Katalysatorphase (208 g aktiviertes DEF) abgetrennt. Die obere Phase enthält 2122 g Behensäurechlorid (99,2 % Ausbeute, bezogen auf eingesetzte technische Behensäure) mit einer Reinheit von 94 % (IR). Die Iodfarbzahl des Säurechlorids beträgt 20. Sowohl das Säurechlorid als auch der Katalysator sind phosgenfrei.

Beispiel 13

In einem thermostatisierten 5-l-Reaktor werden 1120 g (4 mol) Tallölfettsäure (Gemisch einfach und mehrfach ungesättigter $C_{18}$-Carbonsäuren) und 147 g (ca. 0,8 mol) aktiviertes Diethylformamid bei 70°C vorgelegt. Anschließend werden gleichmäßig über einen Zeitraum von 2,0 Stunden unter gutem Rühren 390 g (3,94 mol) Phosgen eingegast. Die Innentemperatur beträgt während der Phosgenzufuhr 72 bis 74°C. Das Abgas der Reaktion wird direkt über einen Wascher abgeleitet. Nach dem Ende der Umsetzung ist der Dampfraum über dem Reaktionsansatz phosgenfrei.

Danach wird 1,0 Stunden bei 72°C nachgerührt, der Rührer abgeschaltet und der Reaktionsansatz auf 20°C abgekühlt. Bei dieser Temperatur wird nach 1,5 Stunden aus dem zweiphasigen Reaktionsansatz die untere Katalysatorphase (141 g aktiviertes DEF) abgetrennt. Die obere Phase enthält 1188 g Tallölfettsäurechlorid (99,5 % Ausbeute, bezogen auf eingesetzte Tallölfettsäure) mit einer Reinheit von 92 % (IR). Die Iodfarbzahl des Säurechlorids beträgt 100. Sowohl das Säurechlorid als auch der Katalysator sind phosgenfrei.

Vergleichsbeispiel A

In einem thermostatisierten Reaktor werden 1946 g (7 mol) technische Stearinsäure bei 60 bis 65°C geschmolzen und 12,8 g (0,175 mol) Dimethylformamid (DMF) zugegeben. Anschließend wird gleichmäßig über einen Zeitraum von 3,0 Stunden unter gutem Rühren Phosgen eingegast, bis das Säurechlorid typgerecht ist. Die Innentemperatur während der Phosgenzufuhr wird zwischen 60 und 65°C gehalten. Die benötigte Phosgenmenge beträgt 940 g (9,5 mol).

Das Säurechlorid enthält Phosgen und wird 12 Stunden bei Raumtemperatur mit trockenem Stickstoff entphosgeniert. Dabei scheidet sich der Katalysator als Feststoff ab. Nach Filtration werden 2025 g (97,6 % Ausbeute) Stearinsäurechlorid in 98 %iger Reinheit erhalten.

Vergleichsbeispiel B analog DE-A-29 50 155

1355 g (5,0 mol) technische Stearinsäure werden bei 60°C geschmolzen und mit 2,7 g (0,0172 mol) Diisobutylformamid versetzt. Die Apparatur besitzt einen Rückflußkühler (Kühlmitteltemperatur: -20°C) mit nachgeschaltetem Wascher. Bei 60 bis 65°C wird unter gutem Rühren gasförmiges Phosgen eingeleitet, bis die Umsetzung der Stearinsäure zum Säurechlorid vollständig ist. Das Phosgen wird so dosiert, daß am Rückflußkühler nur ein äußerst schwacher Phosgenrückfluß auftritt. Es werden 781 g (7,9 mol) Phosgen benötigt. Die Phosgenzugabe dauert 9 Stunden.

Das phosgenhaltige Stearinsäurechlorid wird 3 Stunden bei 60°C mit trockenem Stickstoff entphosgeniert. Der Reaktionsansatz bleibt homogen. Der Austrag an rohem Säurechlorid beträgt 1435 g (maximal mögliche Ausbeute an Stearinsäurechlorid 1448 g) bei einem Reinheitsgrad von 96 % (IR). Die IFZ des braunen Produktes beträgt 100.

**Patentansprüche**

**1.** Verfahren zur Herstellung von Carbonsäurechloriden der allgemeinen Formel I

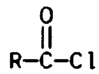

(I),

in der R für $C_8$- bis $C_{30}$-Alkyl, $C_8$- bis $C_{30}$-Alkenyl und $C_8$- bis $C_{30}$-Alkinyl steht, aus Carbonsäuren der allgemeinen Formel II

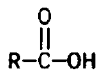

(II),

in der R die oben genannten Bedeutungen hat, und Phosgen, $COCl_2$ (III), in Gegenwart eines Katalysator-Addukts aus Phosgen und N,N-Dialkylformamid der allgemeinen Formel IV

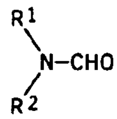

(IV),

in der $R^1$ und $R^2$ unabhängig voneinander $C_1$- bis $C_3$-Alkyl bedeuten, wobei das Reaktionsprodukt I durch Phasentrennung erhalten wird, dadurch gekennzeichnet, daß man II und III in im wesentlichen äquimolaren Mengen verwendet und die das Katalysator-Addukt enthaltene Phase wiederverwendet.

2. Verfahren nach Anspruch 1, dadurch gekennzeichnet, daß man als N,N-Dialkylformamid N,N-Dimethylformamid, Methyl-ethylformamid oder Diethylformamid verwendet.

3. Verfahren nach Anspruch 1, dadurch gekennzeichnet, daß man als N,N-Dialkylformamid N,N-Diethylformamid verwendet.

4. Verfahren nach Anspruch 1, dadurch gekennzeichnet, daß man 5 bis 200 mol.% des Katalysator-Adduktes verwendet.

5. Verfahren nach Anspruch 1, dadurch gekennzeichnet, daß man 10 bis 100 mol.% des Katalysator-Adduktes verwendet.

6. Verfahren nach Anspruch 1, dadurch gekennzeichnet, daß man 10 bis 30 mol.% des Katalysator-Adduktes verwendet.

7. Verfahren nach Anspruch 1, dadurch gekennzeichnet, daß man als Katalysator-Addukt ein 1:1-Addukt, gegebenenfalls in überschüssigem N,N-Dialkylformamid einsetzt.

## Claims

1. A process for the preparation of an acyl chloride of the formula I

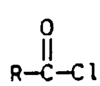

(I)

where R is $C_8$-$C_{30}$-alkyl, $C_8$-$C_{30}$-alkenyl or $C_8$-$C_{30}$-alkynyl, from a carboxylic acid of the formula II

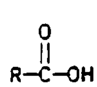

(II)

where R has the abovementioned meanings, and phosgene, $COCl_2$ (III), in the presence of a catalyst adduct of phosgene and N, N-dialkylformamide of the formula IV

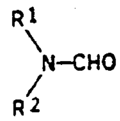

(IV)

where $R^1$ and $R^2$ independently of one another are each $C_1$-$C_3$-alkyl, the reaction product I being obtained by phase separation, wherein II and III are used in essentially equimolar amounts and the phase containing the catalyst adduct is reused.

2. A process as claimed in claim 1, wherein the N,N-dialkylformamide used is N,N-dimethylformamide, methylethylformamide or diethylformamide.

3. A process as claimed in claim 1, wherein the N, N-dialkylformamide used is N,N-diethylformamide.

4. A process as claimed in claim 1, wherein from 5 to 200 mol % of the catalyst adduct are used.

5. A process as claimed in claim 1, wherein from 10 to 100 mol % of the catalyst adduct are used.

6. A process as claimed in claim 1, wherein from 10 to 30 mol % of the catalyst adduct are used.

7. A process as claimed in claim 1, wherein the catalyst adduct used is a 1 : 1 adduct, if necessary in excess N,N-dialkylformamide.

## Revendications

1.- Procédé de préparation de chlorures d'acides carboxyliques de formule générale I

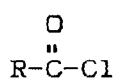

(I)

dans laquelle R est mis pour un radical allyle en $C_8$ à $C_{30}$, alcényle en $C_8$ à $C_{30}$ ou alcynyle en $C_8$ à $C_{30}$, à partir d'acides carboxyliques de formule générale II

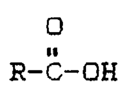

(II)

dans laquelle R a les significations sus-indiquées, et de phosgène $COCl_2$ (III), en présence d'un catalyseur constitué par un produit d'addition de phosgène et de N,N-diallylformamide de formule générale IV

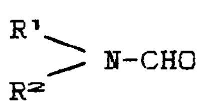

(IV)

dans laquelle $R^1$ et $R^2$ représentent, indépendamment l'un de l'autre, des radicaux allyle en $C_1$ à $C_3$, le produit réactionnel I étant obtenu par séparation de phases, caractérisé en ce qu'on utilise II et III dans des quantités sensiblement équimolaires et en ce qu'on réutilise la phase contenant le produit d'addition catalyseur.

2.- Procédé selon la revendication 1, caractérisé en ce qu'on utilise, comme N,N-dialkylformanide, du N,N-diméthylformamide, du méthyléthylformamide ou du diéthylformamide.

3.- Procédé selon la revendication 1, caractérisé en ce qu'on utilise, comme N,N-dialkylformanide, du N,N-diéthylformamide.

4.- Procédé selon la revendication 1, caractérisé en ce qu'on utilise de 5 à 200% en moles du produit

d'addition catalyseur.

5.- Procédé selon la revendication 1, caractérisé en ce qu'on utilise de 10 à 100% en moles du produit d'addition catalyseur.

6.- Procédé selon la revendication 1, caractérisé en ce qu'on utilise de 10 à 30% en moles du produit d'addition catalyseur.

7.- Procédé selon la revendication 1, caractérisé en ce qu'on utilise, comme produit d'addition catalyseur, un produit d'addition 1:1, éventuellement dans du N,N-diallylformamide en excès.